# EUROPÄISCHE PATENTSCHRIFT

(11) **EP 1 858 447 B1**
(45) Veröffentlichungstag und Bekanntmachung des Hinweises auf die Patenterteilung: **18.08.2010**
(21) Anmeldenummer: 06708462.4
(22) Anmeldetag: 23.02.2006
(51) Int. Cl.: A61F 2/14

(54) **EINRICHTUNG UND VORRICHTUNG ZUM AUSGLEICH EINER LOKALEN VERFORMUNG DER HORNHAUT EINES AUGES**
SYSTEM AND DEVICE FOR COMPENSATING A LOCAL DEFORMATION OF THE CORNEA OF AN EYE
DISPOSITIF DE COMPENSATION D'UNE DEFORMATION LOCALE DE LA CORNEE D'UN OEIL

(30) Priorität: 25.02.2005 DE 102005009259
(43) Veröffentlichungstag der Anmeldung: 28.11.2007
(73) Patentinhaber: Acri. Tec GmbH, 81829 München (DE); Tomalla, Karin, 45149 Essen (DE); Tomalla, Jutta, 45149 Essen (DE)
(72) Erfinder: TOMALLA, Mark, 45478 Mülheim (DE); TOMALLA, Gebhard, 45149 Essen (DE)
(74) Vertreter: Cohausz & Florack
(86) Internationale Anmeldenummer: PCT/EP2006/060199
(87) Internationale Veröffentlichungsnummer: WO 2006/089922

(56) Entgegenhaltungen:
- WO-A-00/25704
- WO-A-00/33729
- WO-A-01/82815
- WO-A-97/49354
- US-A- 6 096 076

## Beschreibung

Die vorliegende Erfindung betrifft eine Einrichtung und eine Vorrichtung zum Ausgleich einer lokalen Verformung der Hornhaut eines Auges.

Eine derartige Einrichtung und eine derartige Vorrichtung werden zur Kompensation einer lokalen Verformung der Hornhaut des Auges, insbesondere des menschlichen Auges, eingesetzt. Sie können aber auch zur Behandlung des Auges eines anderen Lebewesens verwendet werden.

Die Hornhaut eines menschlichen Auges kann aufgrund von Erkrankungen und/oder Verletzungen Dickenminderungen erfahren, die dazu führen, dass unter dem Einfluss des Kammerwasserdruckes die im gesunden Zustand sphärisch geformte Kontur der Hornhaut lokale nach außen oder innen geformte Wölbungen aufweist. Dies führt zu einer Refraktionsänderung mit zum Teil erheblichen Hornhautverkrümmungen (Astigmatismen), die weder durch eine Brille noch durch Kontaktlinsen in zufriedenstellender Weise ausgeglichen werden können. Erhebliche Einschränkungen des Sehvermögens sind die Folge. Eine solche Verformung der Hornhaut ist unter der Bezeichnung Keratokonus oder Keratektasie bekannt.

Die Beseitigung der Hornhautwölbung bzw. Hornhautbeule erfolgt bisher durch von außen eingebrachte Vorschubwerkzeuge, mit denen rein mechanisch und manuell ein annähernd ringförmiger Kanal oder Tunnel im gesunden Bereich der Hornhaut angelegt wird. Der Kanal oder Tunnel dient als Aufnahme zum Einsetzen von Implantaten, die in der Regel aus einem durchsichtigen Kunststoff, wie Polymethylmethacrylat (PMMA), bestehen. Derartige Implantate sind unter der Bezeichnung "INTACS" in der Praxis bekannt.

Bei dem bekannten Verfahren werden beispielsweise zwei in ihrer Längserstreckung bogenförmige Implantate in zwei entsprechende in einer gemeinsamen Ebene liegenden Aufnahmen im gesunden Bereich der Hornhaut eingesetzt. Die Implantate können dabei zum Beispiel einen Winkelbereich von weniger als 180° umfassen und werden üblicherweise derart einander gegenüberliegend in die Hornhaut eingesetzt, dass sie den krankhaft ausgewölbten Hornhautbereich zumindest zum Teil umfassen. Die Implantate üben im in die Hornhaut eingesetzten Zustand eine Zugkraft auf den ausgewölbten Bereich der Hornhaut aus, indem sie sich an einer Wand der jeweiligen Aufnahme abstützen. Dadurch kommt es zu einer Rückformung der Auswölbung.

Das bekannte Verfahren entstammt der Behandlung bei Myopie (Kurzsichtigkeit) und wurde im Jahre 2004 durch die US Food and Drug Administration (FDA) als Verfahren zur Behandlung von Keratokonus- und Keratektasie-Patienten zugelassen.

Derartige Operationsmethoden haben insbesondere den Nachteil, dass eine mehrere Monate dauernde Rekonvaleszenz und Nachsorge des Patienten erforderlich sind. Die aus Schnittwunden resultierenden Narbenauswirkungen und der sich ausbildende Astigmatismus müssen ebenfalls als nachteilig angeführt werden. Außerdem kommt es bei der Präparation oft zu einem Einschleppen von Epithelzellen in die jeweilige Aufnahme, die unerwünschte Ablagerungen an den INTACS erzeugen können. Auch ist die Verletzung der an der Innenseite der Hornhaut verlaufenden Endothelzellen aufgrund der Zug-und Druckbelastung im Rahmen der Durchführung des bekannten Operationsverfahrens nicht zu umgehen.

Bei fortgeschrittenem Befund muss eine komplexe Hornhaut-De-/Reimplantation (perforierende Keratoplastik) erfolgen.

Weiterhin ist bei dieser Operationsmethode nachteilig, dass aufgrund der festdimensionierten Schnittweite keinerlei Variabilität beim Einbringen der Kanäle für die Implantate gegeben ist, wodurch es zu mehr oder weniger ausgeprägter Spannung in der behandelten Hornhaut kommen kann. Mit dem bekannten Verfahren lassen sich die lokale Verformung der Hornhaut und insbesondere die im Bereich der Verformung auftretenden komplexen Spannungsverhältnisse daher oftmals nicht in ausreichend präziser Weise ausgleichen, so dass auch die Verformung nicht in optimaler Weise beseitigt werden kann.

Die WO97/49354 beschreibt die Merkmale des Oberbegriffs des Anspruchs 1.

Der vorliegenden Erfindung lag daher die Aufgabe zugrunde, eine Einrichtung der eingangs genannten Art anzugeben, mit der ein im Vergleich zum Stand der Technik verbesserter Ausgleich einer lokalen Verformung der Hornhaut eines Auges ermöglicht wird. Außerdem soll eine Vorrichtung der eingangs genannten Art angegeben werden, mit der bei verbessertem Ausgleich einer lokalen Hornhautverformung unerwünschte Neben- und/oder Nachwirkungen auf den Patienten minimiert werden können.

In Bezug auf die Einrichtung wird diese Aufgabe gelöst durch eine Einrichtung zum Ausgleich einer lokalen Verformung der Hornhaut eines Auges, mit einem mindestens zwei Implantatsglieder umfassenden Set zum Einsetzen in mindestens eine in die Hornhaut eingebrachte Aufnahme, wobei mindestens zwei Implantatsglieder in unterschiedlichen übereinander liegenden Ebenen angeordnet sind.

Die erfindungsgemäße Einrichtung weist ein Set zum Einsetzen in eine in die Hornhaut eingebrachte Aufnahme auf. Das Set umfasst dabei mindestens zwei Implantatsglieder, die in unterschiedlichen neben und übereinander liegenden Ebenen angeordnet werden. Das Set kann sowohl einstückig ausgebildet sein als auch zwei nicht direkt miteinander verbundene Implantatsglieder umfassen. Die Anordnung der Implantatsglieder in zwei Ebenen bleibt dabei insbesondere nach dem Einsetzen in die Hornhaut erhalten. Die Implantatsglieder sind insbesondere zum Einsetzen in den von der Verformung nicht betroffenen Hornhautbereich vorgesehen. Die Aufnahme, in die die Implantatsglieder jeweils eingesetzt werden, kann dabei insbesondere kanalförmig ausgebildet sein.

Indem erfindungsgemäß mindestens zwei Implantatsglieder in unterschiedlichen Ebenen angeordnet sind, wird den komplexen Spannungsverhältnissen in einer verformten Hornhaut Rechnung getragen. Durch eine derartige Anordnung der Implantatsglieder können in unterschiedlichen Ebenen der Hornhaut durch die Implantatsglieder unterschiedliche Kräfte ausgeübt werden. Auf diese Weise ist es möglich, die in einer verformten Hornhaut vorliegenden Spannungsverhältnisse, und somit die Verformung der Hornhaut, in gegenüber dem Stand der Technik präziserer Weise auszugleichen. Insbesondere können auf diese Weise in verschiedenen Ebenen der Hornhautverformung vorliegende unterschiedliche Spannungsverhältnisse besser ausgeglichen werden.

Dabei kann eine mechanische Beschädigung des gewölbten und dickengeminderten Hornhautbereichs vermieden werden und die lokale Wölbung in der Hornhaut durch insbesondere in dem Umfeld der Wölbung und damit gesunden Bereich in die Hornhaut eingesetzte Implantatsglieder kompensiert werden. Durch eine geeignete Abstimmung von Ausbildung der Aufnahme in der Hornhaut und Ausbildung der in die Aufnahme einzusetzenden Implantatsglieder kann die von dem jeweiligen Implantatsglied auf die Hornhaut, und dabei insbesondere auf den verformten Bereich, ausgeübte Kraft eingestellt werden.

Die Spanneigenschaften der Implantatsglieder können während des Herstellprozesses durch Materialzustands-, Form- und Volumenänderungen bzw. -abweichungen in der Erstreckung des Elements bewirkt sein. Die auf die in die Hornhaut eingebrachte Aufnahme auszuübenden Kräfte können beispielsweise über die Gesamtlängserstreckung der Implantatsglieder oder nur abschnittsweise eingeleitet werden.

Die von den Implantatsgliedern ausgeübten Spannkräfte können z. B. aus bereits integrierten oder durch das Einsetzen in die Hornhaut aufgebauten Spannungen erzeugt werden. Insbesondere können die Spannkräfte aus der geometrischen Dimensionierung von Implantatsglied und zugehöriger Aufnahme herrühren.

Dabei können die Implantatsglieder in den für sie vorgesehenen Aufnahmen konzentrisch oder exzentrisch gelagert sein. Sofern es sich um am Innen- bzw. Außenumfang der Aufnahme anliegende Implantatsglieder handelt, können die Spannkräfte beispielsweise auf eine senkrechte Wand der Hornhautaufnahme wirken.

Gemäß einer bevorzugten Ausgestaltung ist vorgesehen, dass die Implantatsglieder derart ausgebildet sind, dass im in die Hornhaut eingesetzten Zustand mindestens ein Implantatsglied eine Druckkraft und mindestens ein Implantatsglied eine Zugkraft auf den Bereich der lokalen Verformung ausübt. Gemäß dieser Ausgestaltung können die in unterschiedlichen Ebenen angeordneten Implantatsglieder in unterschiedliche, insbesondere entgegengesetzte Richtungen wirkende Kräfte auf die Hornhautverformung ausüben, nämlich Zug- und Druckkräfte. Die in der Verformung bestehenden Spannungsverhältnisse können mit dieser Ausgestaltung noch präziser ausgeglichen werden.

Gemäß einer besonders bevorzugten Ausgestaltung kann das die Druckkraft ausübende Implantatsglied zum Einsetzen in eine distale Ebene der Hornhaut und das die Zugkraft ausübende Implantatsglied zum Einsetzen in eine proximale Ebene der Hornhaut vorgesehen sein. Die distale Ebene ist dabei weiter von dem Augenzentrum entfernt als die proximale Ebene. Es hat sich gezeigt, dass in dem distalen Bereich der verformten Hornhaut eine erhöhte Zugspannung vorliegt, während es in dem proximalen Bereich der verformten Hornhaut zu einer geringeren Zugspannung kommt. Mit dieser Ausgestaltung werden diese Spannungsverhältnisse und damit die Verformung der Hornhaut somit in optimaler Weise ausgeglichen.

In Extremfällen kann es im proximalen Bereich der Hornhaut sogar zu einer Druckspannung kommen. In diesem Fall kann die Hornhaut in dem verformten Bereich eine so genannte "neutrale Faser" aufweisen, oberhalb derer eine Zugspannung und unterhalb derer eine Druckspannung vorliegt. In diesem Fall ist es vorteilhaft, wenn das die Druckkraft ausübende Implantatsglied zum Einsetzen in die Hornhaut oberhalb der neutralen Faser und das die Zugkraft ausübende Implantatsglied zum Einsetzen in die Hornhaut unterhalb der neutralen Faser vorgesehen ist.

Als in der Praxis besonders geeignet hat es sich erwiesen, wenn mindestens eines der Implantatsglieder in Richtung seiner Längserstreckung bogenförmig ausgebildet ist. In ihrer Längserstreckung bogenförmige, insbesondere kreisbogenförmige Implantatsglieder können an die Geometrie der Hornhautverformung, insbesondere ihre oftmals kreisförmige Begrenzung, besonders gut angepasst werden und die ihnen jeweils zugeordneten Kräfte aufbringen. Die bogenförmige Ausbildung erlaubt es darüber hinaus, das Implantatsglied so anzuordnen, dass es die Verformung der Hornhaut mindestens zum Teil umfasst. Eine von dem jeweiligen Implantatsglied auf den verformten Bereich ausgeübte Kraft wirkt so mit großer Gleichmäßigkeit auf den verformten Hornhautbereich. Genauso können insbesondere sämtliche Implantatsglieder bogenförmig ausgebildet sein.

Gemäß einer bevorzugten Lehre kann das bogenförmige Implantatsglied einen Winkelbereich von weniger als 360° umfassen. Ein solches Implantatsglied lässt sich in vereinfachter Weise beginnend mit seinem einen Ende in die in der Hornhaut des Auges vorgesehene Aufnahme einführen. Weiter bevorzugt kann das bogenförmige Implantatsglied einen Winkelbereich von weniger als 180° umfassen. Auf diese Weise ist es möglich, auf gegenüberliegenden Seiten der Hornhautauswölbung jeweils Implantatsglieder vorzusehen. Dabei können zum einen auf einer Seite der Verformung vorgesehene Implantatsglieder in unterschiedlichen Ebenen angeordnet sein. Zusätzlich oder alternativ können aber auch auf gegenüberliegenden Seiten der Verformung angeordnete Implantatsglieder jeweils in unterschiedlichen Ebenen angeordnet sein. Diese Vielzahl von Variationen stellt sicher, dass jedwede Verformung der Hornhaut, beispielsweise auch eine asymmetrische Verformung, präzise ausgeglichen werden kann.

Es kann vorgesehen sein, dass das bogenförmige Implantatsglied eine zweite Krümmung aufweist, deren Krümmungsvektor im Wesentlichen senkrecht zu dem Krümmungsvektor der Bogenkrümmung steht. Gemäß dieser Ausgestaltung weist das Implantatsglied nicht nur in Längsrichtung eine bogenförmige Krümmung, sondern zusätzlich eine zweite Krümmung in eine zu der Bogenkrümmung senkrechte Richtung auf. Auf diese Weise können die Implantatsglieder optimal an die sphärische Kontur der Hornhaut angepasst werden. Auch die zweite Krümmung kann dabei bogenförmig ausgebildet sein. Insbesondere kann die Grundfläche der Implantatsglieder parallel zur Hornhautoberfläche (innen wie außen) verlaufen, damit eine Verformung der Hornhaut quer zur Hornhautverkrümmung durch ein Durchdrücken der Implantatsglieder vermieden wird. Selbstverständlich kann auch die für ein jeweiliges Implantatsglied vorgesehene Aufnahme eine entsprechende zweite Krümmung aufweisen. Auch kann das Implantatsglied bzw. die zugehörige Aufnahme natürlich auch noch weitere Krümmungen in weitere Richtungen aufweisen.

Da es das Ziel ist, den erkrankten dünnwandigen und nach außen geformten Hornhautbereich so rückzuformen, dass dieser an die übrige sphärisch gekrümmte Hornhautoberfläche optimal angepasst ist, ist es günstig, mehrere Stellgrößen, wie das Implantatsglied und die Aufnahme, heranzuziehen, um hierdurch adäquate Spannungen und Verformungswege gezielt erzeugen zu können. Insbesondere kann es dabei zweckmäßig sein, lokal und abgestimmt geometrische Abweichungen an Implantatsglied-und/oder Form der Aufnahme vorzunehmen. Sämtliche der möglichen Anpassungen der Geometrie der Implantatsglieder können natürlich alternativ oder zusätzlich auch an der Form der Aufnahme in der Hornhaut vorgenommen werden.

Gemäß einer Ausgestaltung kann mindestens eines der Implantatsglieder mäanderförmig ausgebildet sein. Durch diese Ausgestaltung ist es je nach Ausbildung des Mäanders möglich, gezielt an die Hornhautverformung angepasste Kräfte durch das Implantatsglied in die Hornhaut einzubringen. Insbesondere führen dabei die Bereiche des Implantatsglieds, in denen der Mäander Wendepunkte aufweist, aufgrund eines verstärkten Abstützens an der Aufnahme zu einer erhöhten Krafteinleitung. Durch eine geeignete Ausgestaltung des Mäanders und der zugehörigen Aufnahme lassen sich somit die Krafteinleitung in die Hornhaut individuell einstellen und so ein noch flexiblerer Ausgleich der Verformung der Hornhaut erreichen. Dabei kann der Mäander insbesondere unterschiedliche Anzahlen, unterschiedliche Abstände und/oder unterschiedliche Positionen der Wechsel- oder Wendepunkte und/oder Unterschiede in den äußeren wie auch inneren Radien, insbesondere den radialen Differenzen zwischen dem Größt- und Kleinstdurchmesser des Mäanders aufweisen (Frequenz und Amplitude).

Eine Flexibilisierung beim Einleiten von Ausgleichskräften in die Hornhaut kann auch erreicht werden, wenn mindestens eines der Implantatsglieder polygonförmig ausgebildet ist. Auch mit einer solchen Ausgestaltung kann das Implantatsglied in seiner Wirkung optimal an die Ausprägung der jeweiligen Hornhautverformung angepasst werden. Lediglich beispielhaft für eine geeignete Polygonform wird ein Sechseck genannt.

Eine individuelle Anpassung der Wirkung der Einrichtung kann auch dadurch erreicht werden, dass mindestens eines der Implantatsglieder entlang seiner Längserstreckung unterschiedliche Festigkeits- und/oder Elastizitätswerte aufweist. Die unterschiedlichen Festigkeits- und/oder Elastizitätswerte führen dazu, dass die von dem Implantatsglied auf die Hornhaut ausgeübten Spannkräfte je nach Festigkeit und Elastizität des jeweiligen Gliedabschnitts variieren. So kann auch auf diese Weise eine individuelle Anpassung des Implantatsglieds an die auszugleichende Verformung der Hornhaut erreicht werden.

Eine weitere Ausgestaltung sieht vor, dass mindestens eines der Implantatsglieder einen keilförmigen Querschnitt aufweist. Durch eine entsprechende Ausbildung des Keilquerschnitts kann die Krafteinleitung besonderes gezielt erfolgen. Dabei sollte die Keilschneide in die jeweils beabsichtigte Kraftwirkrichtung zeigen, da in dieser Richtung von dem keilförmigen Implantatsglied eine verhältnismäßig große Kraft ausgeübt wird. Das Implantatsglied kann so beispielsweise einen dreieckigen Querschnitt aufweisen.

Die Ausbildung des Sets mit zwei nicht direkt miteinander verbundenen Implantatsgliedern hat den Vorteil, dass robuste Elemente zur Verfügung stehen, die sich einwandfrei handhaben lassen.

Eine einstückige Ausbildung des erfindungsgemäßen Sets hat demgegenüber andere Vorteile. So wird bei einem einstückigen Set der Ausgleich der Hornhautverformung durch Einsetzen nur eines Bauteils in die Hornhaut erreicht. Es ist somit nicht erforderlich, zwei Aufnahmen in die Hornhaut einzubringen und zwei Implantatsglieder in der Hornhaut aufwändig zueinander zu positionieren, um eine gewünschte Krafteinleitung in die Hornhaut zu erreichen. Daher sieht eine alternative Ausgestaltung der Erfindung vor, dass das Set einstückig ausgebildet sein kann. In diesem Fall sind die Implantatsglieder des Sets also einstückig miteinander verbunden.

Bei einem einstückig ausgebildeten Set können die Implantatsglieder im Wesentlichen parallel zueinander ausgerichtet sein und durch einen Verbindungssteg miteinander verbunden sein, wobei der Verbindungssteg mit seinem einen Stegende mit einem in Richtung der Quererstreckung der Implantatsglieder vorderen Ende des einen Implantatsglieds verbunden ist und mit seinem anderen Stegende mit einem in Richtung der Quererstreckung der Implantatsglieder hinteren Ende des anderen Implantatsglieds verbunden ist. Mit dieser Ausgestaltung lässt sich also beispielsweise ein Z-förmiger Querschnitt des Sets erzeugen. Die Z-Form kann dabei bereits vor dem Einsetzen in die Hornhaut vorliegen. Es kann aber auch vorgesehen sein, dass die Z-Form des Sets erst durch eine Verspannung beim Einsetzen in die in der Hornhaut vorgesehene Aufnahme entsteht.

In jedem Fall kann mit einem einen Z-förmigen Querschnitt aufweisenden Set durch eine entsprechende Verspannung des Sets in der in der Hornhaut vorgesehenen Aufnahme mit nur einem Bauteil sowohl eine Druckkraft als auch eine Zugkraft auf den verformten Bereich der Hornhaut ausgeübt werden. Dabei kann das im eingesetzten Zustand den von der Hornhautverformung weggerichteten Schenkel der Z-Form bildende Implantatsglied eine Zugkraft ausüben, während das den zu der Verformung hingerichteten Schenkel der Z-Form bildende Implantatsglied eine Druckkraft ausüben kann. Insbesondere kann dabei vorgesehen sein, dass das die Druckkraft ausübende Implantatsglied des Sets zum Einsetzen in eine distale Ebene der Hornhaut vorgesehen ist, während das die Zugkraft ausübende Implantatsglied zum Einsetzen in eine proximale Ebene vorgesehen ist.

Gemäß einer besonders praxisgerechten Ausgestaltung kann mindestens eines der Implantatsglieder einen Bereich aufweisen, der mittels magnetischer Kräfte manipuliert werden kann. Das Implantatsglied weist in diesem Fall einen Bereich auf, über den das Implantatsglied mittels magnetischer Kräfte bewegt werden kann. Es ist auf diese Weise in besonders einfacher Weise möglich, das Implantatsglied in die Aufnahme in der Hornhaut einzuführen, indem das Implantatsglied mittels eines geeigneten magnetischen Werkzeugs geführt wird. Die mit dem Eingriff verbundenen Belastungen für das Auge sind bei dieser Ausgestaltung minimiert, da beim Einsetzen des Implantatsglieds in die Hornhaut kein direkter Kontakt mit dem Implantatsglied erforderlich ist.

Als Werkstoff für die Implantatsglieder bzw. das Set kommt vorrangig ein transparenter Werkstoff wie PMMA in Frage. Dieser Werkstoff hat sich in der Praxis bewährt. Insbesondere verbindet er gute Spanneigenschaften mit einer guten Verträglichkeit durch das Auge. Neben dem vorrangig verwendeten Werkstoff PMMA können aber auch andere transparente und körpergerechte Werkstoffe wie zum Beispiel solche mit einem irreversiblen, über die gesamte Gebrauchsdauer des Implantatsglieds anhaltenden Formänderungseffekt (Memory) verwendet werden.

Bei Verwendung von transparenten Werkstoffen, beispielsweise das bereits genannte PMMA, für die Implantatsglieder kommen bevorzugt solche in Betracht, die ein E-Modul von ca. 600 MPa und größer aufweisen, da eine nennenswerte Eigensteifigkeit notwendig ist, um die Hornhaut selbst rückformstabil halten zu können.

Ferner kommen Werkstoffe in Frage, die im Implantierzustand bereits integrierte Eigenspannungen besitzen oder die Fähigkeit zum Aufbau von Eigenspannungen durch Fremdeinwirkung.

Die für die erfindungsgemäßen Implantatsglieder verwendeten Werkstoffe sollten dabei im Laufe ihrer Lebensdauer ihr Eigenvolumen nicht verändern, also insbesondere nicht quellen, um eine gleichmäßige Wirkung der Implantatsglieder zu gewährleisten.

Mit derartigen Materialien kann in besonders individueller Weise die jeweils erforderliche Ausbildung, insbesondere Geometrie, gewählt werden, ohne dass die Implantatsglieder speziell für eine Behandlung hergestellt werden müssen. Es ist aber auch denkbar, dass ein Material für die Implantatsglieder vorgesehen ist, das einen reversiblen Formänderungseffekt (Memory) besitzt. In diesem Fall ist es möglich, das Implantatsglied beispielsweise bei einer längere Zeit nach der Behandlung erfolgenden Veränderung der Hornhautgeometrie an die nun vorliegende Geometrie anzupassen und somit weiterzubenutzen.

Die erfindungsgemäße Aufgabe wird außerdem gelöst durch eine Vorrichtung zum Ausgleich einer lokalen Verformung der Hornhaut eines Auges, mit einer Einrichtung zum Erfassen von Eigenschaftsdaten der lokalen Verformung, mit einer Einrichtung zum Simulieren des Ausgleichs der Verformung der Hornhaut, mit der Maßgabe, dass mindestens zwei Implantatsglieder in mindestens eine in die Hornhaut eingebrachte Aufnahme eingesetzt werden, wobei im in die Hornhaut eingesetzten Zustand ein Implantatsglied eine Zugkraft und ein Implantatsglied eine Druckkraft auf den Bereich der lokalen Verformung ausübt, mit einer Einrichtung zum Auswählen einer geeigneten Kombination einer in die Hornhaut einzubringenden Aufnahme und einer in die Aufnahme einzusetzenden, erfindungsgemäß ausgebildeten Einrichtung, unter Berücksichtigung der Ergebnisse der Simulation, und mit einer Einrichtung zum Einbringen der Aufnahme in die Hornhaut. Die Aufnahme kann dabei insbesondere kanalförmig ausgebildet sein.

Um mit der technisch möglichen hohen Maßgenauigkeit in der Herstellung der Hornhautaufnahme und deren Lokalisation auch das optimale operative Ergebnis zu erzielen, sind Vorkehrungen vor der Operation zu treffen, wie z. B. Datenerfassung, Simulation und Optimierung.

Dazu weist die Vorrichtung eine Einrichtung zum Erfassen von Eigenschaftsdaten auf, mit der die vor dem Ausgleich der lokalen Verformung vorliegenden Daten ermittelt werden. Sie dienen als Basis für die weitere Vorgehensweise. Die Einrichtung zum Simulieren des Ausgleichs der Verformung dient dazu, die Auswirkungen der Kompensation der Hornhautwölbung (ideale Hornhautwölbung) auf das gesunde Hornhautumfeld zu ermitteln, um die Lage der Aufnahme für die betreffenden Stabilisierungsimplantatsglieder effektiv und sicher zu bestimmen. Dabei erfolgt die Simulation unter der Maßgabe, dass mindestens zwei Implantatsglieder in mindestens eine in die Hornhaut eingebrachte Aufnahme eingesetzt werden, wobei im in die Hornhaut eingesetzten Zustand ein Implantatsglied eine Zugkraft und ein Implantatsglied eine Druckkraft auf den Bereich der lokalen Verformung ausübt. Diese Maßgabe stellt einen präzisen Ausgleich der Hornhautverformung sicher.

Dabei kann insbesondere eine Einrichtung vorgesehen sein, die ein Optimierungs- bzw. Simulationsmodell für das Kompensieren der Hornhautverformung erstellt. Der hierfür zu nutzende Hornhautbereich liegt üblicherweise auf einem Durchmesser von ca. 8 mm bezogen auf die Augenachse (Mittelpunkt). Hier lassen sich ring- oder teilringförmige Kanäle bzw. Tunnel als Aufnahme in die Hornhaut einbringen und in diese entsprechende Implantatsglieder setzen. Je nach Befund können die Aufnahmen und Implantate konzentrisch oder exzentrisch gelagert sein.

Auch bei der Simulation der Wirkdurchmesser wird in diesem Bereich angesetzt. Ein Quadrantenkreis wird so gelegt, dass die Hornhautwölbung in einem Quadranten liegt. Bezogen auf die Aufnahme- und Implantatsgliedkombinationen und -variationen werden radiale Kraftwirklinien für Zug und/oder Druck in dem jeweiligen Quadranten festgelegt und ihre Größe und deren Wirkung auf die Kompensation der Wölbung ermittelt. Ebenso wesentlich für die Simulation wie die Kenntnis der vorbeschriebenen Größen ist die der Wege für die Rückverformung der Hornhautverformung.

Nach Vorlage der Ergebnisse von Wirkrichtungen und Wirkgrößen in Kraft und Weg wird die am besten geeignete Kombination von Kanal- und Implantatausführung gewählt und umgesetzt. Dazu dient die Einrichtung zum Auswählen einer geeigneten Kombination einer in die Hornhaut einzubringenden Aufnahme und einer in die Aufnahme einzusetzenden, erfindungsgemäß ausgebildeten Einrichtung, unter Berücksichtigung der Ergebnisse der Simulation. Auf Grundlage der Ergebnisse der Simulation wird die für den Ausgleich der jeweiligen Hornhautverformung optimale Kombination von Aufnahme und erfindungsgemäßer Einrichtung zum Ausgleich einer lokalen Hornhautverformung ausgewählt. Schließlich ist eine Einrichtung zum Einbringen der für die jeweils ausgewählte Einrichtung geeigneten Aufnahme in die Hornhaut vorgesehen. In diese Aufnahme wird die Einrichtung anschließend eingesetzt.

Mit der erfindungsgemäßen Vorrichtung ist es möglich, unerwünschte Nach- und Nebenwirkungen des Eingriffs in das Auge bereits im Zuge der Simulation zu erkennen und dementsprechend geeignete Aufnahmen und Einrichtungen auszuwählen, um derartige Nach- und Nebenwirkungen zu minimieren. Gleichzeitig wird ein optimaler Ausgleich der Hornhautverformung durch die erfindungsgemäße Einrichtung zum Ausgleich der Verformung erreicht.

Bei den von der Einrichtung zum Erfassen der Eigenschaftsdaten ermittelten Daten kann es sich insbesondere um Daten bezüglich der Hornhautdicke, -elastizität, -duktilität und/oder -festigkeit in dem verformten Bereich (Endothelmikroskopie) und/oder Daten bezüglich der Geometrie und/oder der Position der lokalen Verformung, insbesondere in Bezug auf die Augenachse und/oder den gesunden Hornhautbereich, handeln. Alle diese Daten sind von Relevanz für die Auswahl von geeigneten Aufnahmen und Implantatsgliedern. Im Anschluss an die Topografie und Bestimmung der geometrischen sowie materialspezifischen Daten der Hornhaut wird dann ein Optimierungs- bzw. Simulationsmodell für das Kompensieren der Hornhautverformung erstellt.

Auch der nicht verformte Bereich der Hornhaut kann untersucht werden, zum einen um unerwünschte Nebenwirkungen auf den gesunden Bereich im Zuge der Behandlung zu vermeiden, aber auch um die erforderlichen und für die Hornhaut verträglichen einzubringenden Kräfte zu ermitteln. Dazu kann die Vorrichtung eine Einrichtung zum Erfassen der Eigenschaftsdaten des nicht verformten Bereichs der Hornhaut aufweisen. Sämtliche für den verformten Bereich erfassten Eigenschaftsdaten können dabei auch für den gesunden Bereich der Hornhaut erfasst werden. Beispielsweise kann dabei auch die sphärische Ausbildung der Hornhaut in dem gesunden Bereich erfasst werden.

Die Vorrichtung kann weiterhin eine Einrichtung zum Markieren des für das Einbringen der Aufnahme vorgesehenen Bereichs der Hornhaut vor dem Einbringen der Aufnahme aufweisen. Mittels dieser Einrichtung wird also nach der Auswahl einer geeigneten Aufnahme und deren Position in der Hornhaut der mit der Aufnahme zu versehende Hornhautbereich markiert, bevor die Aufnahme eingebracht wird. Das Zwischenschalten eines solchen Markierungsvorgangs erfolgt für den Fall, dass es bei dem Andocken der Einrichtung zum Einbringen der Aufnahme an das zu operierende Auge zu nicht vernachlässigbaren Abweichungen der Hornhautkontur kommt. Insbesondere wenn die Einrichtung zum Einbringen der Aufnahme mit einem Adapter an das Auge angedockt wird, können Abweichungen der Hornhautkontur aus den Druckverhältnissen zwischen dem im Adapter aufzubauenden Unterdruck und dem bei der Auflage des Adapters auf das Auge notwendigen Anpressdruck resultieren.

Die Einrichtung zum Markieren kann beispielsweise die sich aus der Simulationsphase ergebenden Lage- und Formdaten auf den normalen Istzustand des Auges derart übertragen, dass z. B. mittels einer der Hornhaut bei kompensierter Hornhautverformung angepassten, vorzugsweise aus PMMA bestehenden Schablone die in idealer Weise dem ausgewählten Implantatsglied entsprechenden Koordinaten für den Verlauf der Aufnahme auf die Hornhaut appliziert werden. Eine entsprechende Lösung lässt sich mit einem im Excimerlaserbereich integrierten biometrisch exakten Erkennungssystem erreichen.

Erfolgt anschließend das Andocken der Einrichtung zum Einbringen der Aufnahme an das Auge, so lässt sich überprüfen, inwieweit der ideale Verlauf der Aufnahme erreicht wird bzw. die Einrichtung zum Einbringen der Aufnahme korrigiert werden muss, um diesen sicherzustellen.

Die Einhaltung dieser Bedingung ist wichtig, denn ihre Nichteinhaltung beeinträchtigt direkt den Erfolg der Operation, indem die ausgewählten Implantatsglieder die vorgesehene Funktion nicht erfüllen können. So führt z. B. das Einbringen der Aufnahme bei zu stark gekrümmter Hornhaut dazu, dass beispielsweise ein Solldurchmesser einer Aufnahme zu groß und bei zu flach gedrückter Hornhaut ein Solldurchmesser einer Aufnahme zu klein ausfällt. Passungenauigkeiten führen nicht nur zu Funktions-, sondern auch zu Implantationsproblemen und deren unerwünschten Folgen.

Die Einrichtung zum Einbringen der Aufnahme kann gemäß einer bevorzugten Ausgestaltung einen auf das Auge aufzusetzenden Adapter zum Fixieren des Auges während des Einbringens der Aufnahme aufweisen. Für die Implantierung des Sets kann es erleichternd sein, wenn mit einem Adapter der Bereich der Hornhaut, in dem nicht implantiert wird, stützend in Sollform gehalten wird. Ein solcher Adapter kann beispielsweise ähnlich einem an sich bekannten Laser-Adapter, der auf das Patienten-Interface (PI) abgestimmt ist, ausgebildet sein.

Der Adapter kann als kegelstumpfförmiger Trichter ausgeführt sein. Der hier vorgeschlagene Adapter kann ähnlich an sich bekannten Adaptern für diese Zwecke dimensioniert sein. Er besitzt jedoch an seinem Umfang eine so große Öffnung, dass das Einbringen des Implantatsglieds in die Aufnahme über den Aufnahmeschnitt störungsfrei bei aufgesetztem Adapter durchgeführt werden kann. Um dem Operateur beste Sicht auf das Operationsfeld zu bieten, eignet sich als Werkstoff für diesen Adapter insbesondere ein voll transparentes Material wie z. B. PMMA.

Dabei sollte die Eingriffsöffnung in dem Adapter zum Einbringen des Implantatsglieds einerseits so groß sein, dass eine einwandfreie Implantation sichergestellt ist. Andererseits ist eine möglichst große Kontaktfläche zwischen Adapter und Hornhaut wünschenswert, um die Hornhautform ausreichend stabilisieren zu können. In der Praxis muss ein geeigneter Kompromiss zwischen diesen beiden Zielen gefunden werden.

Die Einrichtung zum Einbringen der Aufnahme in die Hornhaut kann einen Laser, insbesondere einen Femtosekundenlaser, aufweisen. Mit Lasern ist aufgrund ihrer guten Fokussierbarkeit ein besonders genaues Einbringen der Aufnahme in die Hornhaut möglich. Dabei sind ebenfalls aufgrund der guten Fokussierbarkeit die Auswirkungen durch Energieeinkopplung in das dem Laserfokus beim Einbringen der Aufnahme benachbarte Gewebe minimal. Unerwünschte Auswirkungen auf das Hornhautgewebe können so weitgehend vermieden werden. Femtosekundenlaser sind gepulste Laser, deren Pulsdauern im Bereich von Femtosekunden liegen. Aufgrund der kurzen Pulsdauer derartig hochfrequent gepulster Lasertypen wird Energie von dem Laser nur sehr kurzzeitig in das bearbeitete Gewebe eingekoppelt. Es kommt daher zu keiner wesentlichen Ausbreitung von eingekoppelter Energie in benachbartes Gewebe. Unerwünschte Auswirkungen auf das zu dem für das Einbringen der Aufnahme vorgesehenen Gewebe benachbarte Hornhautgewebe durch Erwärmung werden so weiter minimiert.

Nachfolgend wird die Erfindung anhand von in einer Zeichnung dargestellten Ausführungsbeispielen näher erläutert. Es zeigen:
- Fig. 1: einen teilweisen Querschnitt der Hornhaut eines menschlichen Auges mit einer in die Hornhaut eingesetzten erfindungsgemäßen Einrichtung gemäß einem ersten Ausführungsbeispiel,
- Fig. 2: einen Ausschnitt eines erfindungsgemäßen Implantatsglieds gemäß einem weiteren Ausführungsbeispiel in einer Draufsicht,
- Fig. 3: einen teilweisen Querschnitt der Hornhaut eines menschlichen Auges mit einer in die Hornhaut eingesetzten erfindungsgemäßen Einrichtung gemäß einem weiteren Ausführungsbeispiel,
- Fig. 4: einen teilweisen Querschnitt der Hornhaut eines menschlichen Auges mit einer gemäß einem ersten Ausführungsbeispiel ausgeglichenen Hornhautverformung, und
- Fig. 5: einen teilweisen Querschnitt der Hornhaut eines menschlichen Auges mit einer gemäß einem weiteren Ausführungsbeispiel ausgeglichenen Hornhautverformung.

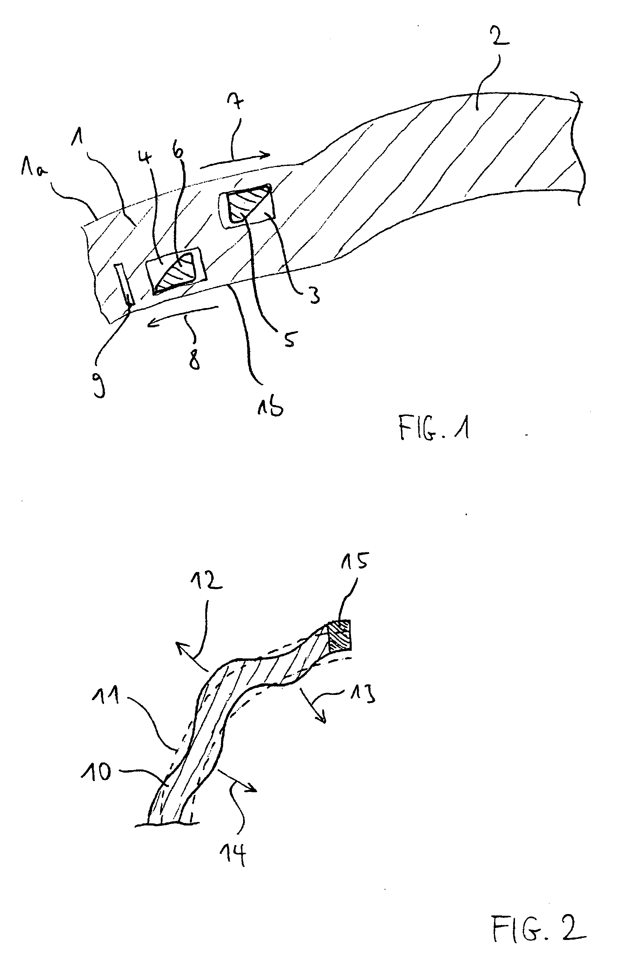

In Figur 1 ist ein Ausschnitt der Hornhaut 1 eines menschlichen Auges in einem Querschnitt dargestellt. Die Hornhaut weist eine distale Oberfläche 1a und eine der Netzhaut des Auges zugewandte proximale Oberfläche 1b auf. In der Hornhaut 1 hat sich eine lokale Verformung 2 in Form einer kreisförmig begrenzten Auswölbung gebildet. In den nicht verformten, gesunden Bereich der Hornhaut 1 sind zwei kanalförmige Aufnahmen 3, 4 mittels eines Femtosekundenlasers eingebracht.

Die Aufnahmen 3, 4 weisen einen rechteckigen Querschnitt auf und sind in ihrer Längserstreckung kreisbogenförmig ausgebildet. Sie umfassen jeweils einen Winkelbereich von weniger als 180°. Der Krümmungsmittelpunkt der Aufnahmen 3, 4 liegt dabei etwa auf einer durch das Zentrum der Verformung 2 verlaufenden, nicht dargestellten Achse. Die Aufnahme 3 ist in eine distale Ebene der Hornhaut 1 und die Aufnahme 4 in eine proximale Ebene der Hornhaut 1 eingebracht.

Zum Ausgleich der Verformung 2 der Hornhaut 1 sind in die Aufnahmen 3, 4 jeweils aus PMMA bestehende Implantatsglieder 5, 6 eingesetzt. Die Implantatsglieder 5, 6 bilden ein in die Hornhaut 1 eingesetztes Set, wobei die Implantatsglieder 5, 6 in unterschiedlichen durch die Aufnahmen 3, 4 definierten Ebenen angeordnet sind. Die Implantatsglieder 5, 6 weisen wie die Aufnahmen 3, 4 eine in ihrer Längserstreckung bogenförmige Form auf und umfassen einen Winkelbereich von weniger als 180°. Die Implantatsglieder 5, 6 besitzen jeweils einen keilförmigen Querschnitt. Dabei ist die Keilschneide des in die distale Ebene der Hornhaut 1 eingesetzten Implantatsglieds 5 in Richtung der Verformung 2 gerichtet, während die Keilschneide des in die proximale Ebene der Hornhaut 1 eingesetzten Implantatsglieds 6 von der Verformung 2 weggerichtet ist. Das in die distale Ebene eingesetzte Implantatsglied 5 besitzt eine geringere Krümmung als die zugehörige Aufnahme 3, während das in die proximale Ebene eingesetzte Implantatsglied 6 eine größere Krümmung besitzt als die zugehörige Aufnahme 4.

Beim Einsetzen der Implantatsglieder 5, 6 in die zugehörigen Aufnahmen 3, 4 kommt es dementsprechend zu einer Verspannung der Implantatsglieder 5, 6 in der Hornhaut 1. Aufgrund der jeweiligen Krümmungsverhältnisse von Implantatsglied 5, 6 zu Aufnahme 3, 4 führt die Verspannung dazu, dass das in die distale Ebene eingesetzte Implantatsglied 5 im Wesentlichen eine Druckkraft auf den Bereich der lokalen Verformung 2 der Hornhaut 1 ausübt, während das in die proximale Ebene eingesetzte Implantatsglied 6 im Wesentlichen eine Zugkraft auf den Bereich der lokalen Verformung 2 der Hornhaut 1 erzeugt. Die von den Implantatsgliedern 5, 6 ausgeübten Druck- bzw. Zugkräfte sind in Figur 1 durch Pfeile 7, 8 veranschaulicht. Auf der nicht dargestellten gegenüberliegenden Seite der Verformung 2 sind zwei entsprechende Implantatsglieder in entsprechende Aufnahmen in die Hornhaut 1 eingesetzt.

Aufgrund der von den Implantatsgliedern in die Hornhaut 1 und insbesondere in den Bereich der Verformung 2 eingeleiteten Kräfte kommt es zu der gewünschten Rückformung der lokalen Verformung 2.

Die Implantatsglieder 5, 6 können eine zweite Krümmung aufweisen, deren Krümmungsvektor im Wesentlichen senkrecht zu dem Krümmungsvektor der in in die Zeichnungsebene gerichteten Längsrichtung gerichtete Bogenkrümmung steht. Auf diese Weise ist es möglich, die Implantatsglieder 5, 6 an die im allgemeinen sphärische Ausbildung der Hornhaut 1 anzupassen, so dass es nicht zu unerwünschten Spannungen beim Einsetzen der Implantatsglieder 5, 6 kommt. Das Ausmaß an Rückverformung der Hornhaut kann zusätzlich dadurch vergrößert werden, dass beispielsweise das Implantatsglied 6 für den proximalen Bereich konkav gewölbte, hier nicht sichtbare Rundungen auf der Grundfläche aufweist und der Kanal 4 im Querschnitt dreiecksförmig ausgeführt ist. Die Rundungen können dabei gleichmäßig über die Länge des Implantatsgliedes 6 verteilt sein oder auf bestimmte Abschnitte beschränkt werden, um lokal eine besondere Ausgleichswirkung zu erzielen.

Um der Hornhaut 1 eine Möglichkeit zum Ausgleich einer eventuellen Komprimierung durch die eingesetzten Implantatsglieder 5, 6 zu geben, ist eine Entlastungskammer 9 in die Hornhaut 1 eingebracht. Die Entlastungskammer 9 verläuft auf der der Verformung 2 abgewandten Seite der Aufnahme 4 im Wesentlichen parallel zu der Aufnahme 4.

In Figur 2 ist ein Ausschnitt eines Implantatsglieds 10 gemäß einem weiteren Ausführungsbeispiel in Draufsicht dargestellt. Das Implantatsglied 10 verläuft über einen Winkelbereich von weniger als 180° bogenförmig und ist mäanderförmig ausgebildet.

An seinem einen Ende weist das Implantatsglied 10 einen magnetempfindlichen Bereich 15 auf, der mittels magnetischer Kräfte manipuliert werden kann. Es ist somit möglich, das Implantatsglied 10 mittels beispielsweise von einem Permanentmagneten auf den Bereich 15 ausgeübter magnetischer Kräfte in eine für das Implantatsglied 10 vorgesehene Aufnahme zu führen, ohne dass ein direkter Kontakt mit dem Implantatsglied 10 zum Einsetzen in die Aufnahme notwendig wäre. Beeinträchtigungen des Auges im Zuge des Einsetzens des Implantatsglieds 10 werden auf diese Weise weitgehend vermieden.

Wird das Implantatsglied 10 in eine bogenförmige Aufnahme eingesetzt, die nicht mäanderförmig ausgebildet ist, wie die in Figur 2 gestrichelt dargestellte Aufnahme 11, so wird von dem Implantatsglied 10 je nach Form des Mäanders eine unterschiedliche Kraft in die Hornhaut eingeleitet. Dabei ist ein in Figur 2 nicht dargestelltes zweites Implantatsglied vorgesehen, das in eine in einer von der Ebene der Aufnahme 11 unterschiedlichen Ebene angeordnete zweite Aufnahme eingesetzt wird. Das zweite Implantatsglied und die zweite Aufnahme können dabei in ihrer Ausbildung im Wesentlichen dem ersten Implantatsglied 10 und der ersten Aufnahme 11 entsprechen. Die Implantatsglieder und/oder die Aufnahmen können aber auch unterschiedlich ausgebildet sein. Selbstverständlich können noch weitere Implantatsglieder zum Einsetzen in die Hornhaut vorgesehen sein.

In den Bereichen, in denen das mäanderförmige Implantatsglied 10 eine stärkere Krümmung aufweist als die Aufnahme 11, übt das Implantatsglied 10 im in die Hornhaut eingesetzten Zustand eine von dem Zentrum der nicht dargestellten lokalen Verformung der Hornhaut weggerichtete Zugkraft auf den verformten Bereich der Hornhaut aus. Dies ist in Figur 2 durch den Pfeil 12 veranschaulicht. In den Bereichen hingegen, in denen das mäanderförmige Implantatsglied 10 in Bezug auf die Aufnahme 11 eine geringere oder sogar entgegengesetzte Krümmung aufweist, übt das Implantatsglied 10 im in die Hornhaut eingesetzten Zustand eine zum Zentrum der nicht dargestellten lokalen Verformung der Hornhaut gerichtete Druckkraft aus. Dies ist in Figur 2 durch die Pfeile 13, 14 veranschaulicht. Das in Figur 2 nicht dargestellte zweite Implantatsglied übt im in die Hornhaut eingesetzten Zustand entsprechende Kräfte auf die Hornhaut aus. Dabei wird durch das Zusammenwirken der von den Implantatsgliedern in unterschiedlichen Ebenen in die Hornhaut eingeleiteten Kräfte eine besonders präzise Rückformung der lokalen Hornhautverformung erreicht. Das Ergebnis der Rückformung kann durch eine entsprechende Ausbildung der Mäander in besonders gezielter Weise beeinflusst werden. Diese Ausgestaltung ist ein Beispiel für eine grundsätzlich punktuelle bzw. lokale Krafteinleitung für sowohl Zug- als auch Druckkräfte.

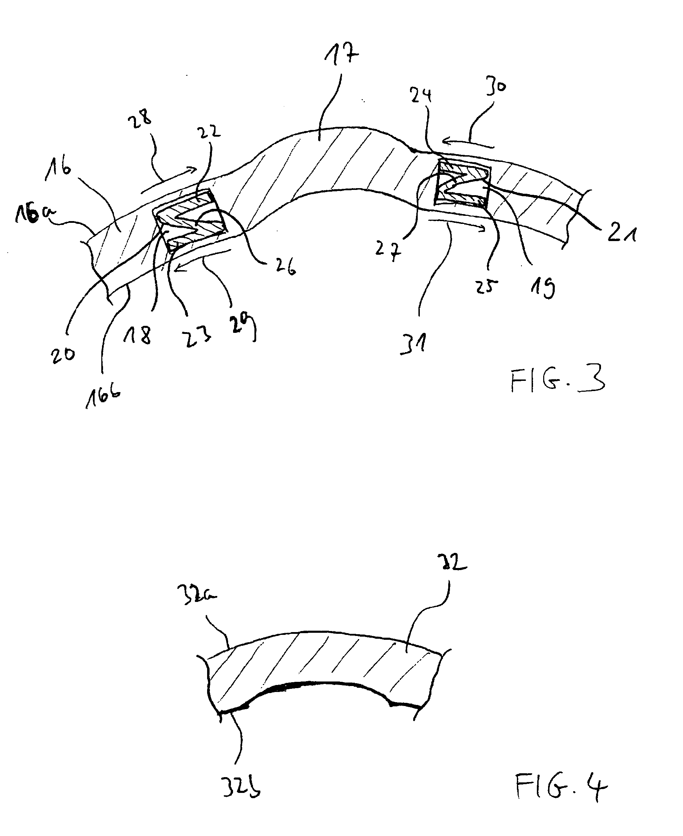

In Figur 3 ist ein Ausschnitt der Hornhaut 16 eines menschlichen Auges in einem Querschnitt dargestellt. Wiederum besitzt die Hornhaut 16 eine distale Oberfläche 16a und eine der Netzhaut des Auges zugewandte proximale Oberfläche 16b.

An der Hornhaut 16 ist eine lokale Verformung 17 in Form einer kreisförmig begrenzten Auswölbung ausgebildet. Beiderseits der Verformung 17 sind in den gesunden Bereich der Hornhaut 16 zwei Aufnahmen 18, 19 eingebracht.

Die kanalförmigen Aufnahmen 18, 19 weisen einen rechteckigen Querschnitt auf und sind in ihrer Längserstreckung bogenförmig, insbesondere kreisbogenförmig ausgebildet. Sie umfassen jeweils einen Winkelbereich von weniger als 180°. Der Krümmungsmittelpunkt der Aufnahmen 18, 19 liegt dabei etwa auf einer durch das Zentrum der Verformung 17 verlaufenden nicht dargestellten Achse.

Zum Ausgleich der Verformung 17 sind in die Aufnahmen 18 und 19 zwei jeweils einstückig ausgebildete Sets 20, 21 eingesetzt. Die Sets 20, 21 umfassen jeweils zwei Implantatsglieder 22, 23, 24, 25. Die Implantatsglieder eines Sets 20, 21 sind im Wesentlichen parallel zueinander ausgerichtet und jeweils durch einen Verbindungssteg 26, 27 miteinander verbunden. Der Verbindungssteg 26, 27 ist mit seinem einen Stegende mit einem in Richtung der Quererstreckung der Implantatsglieder 22, 23, 24, 25 vorderen Ende des einen Implantatsglieds 22, 24 und mit seinem anderen Stegende mit einem in Richtung der Quererstreckung der Implantatsglieder 22, 23, 24, 25 hinteren Ende des anderen Implantatsglieds 23, 25 angeschlossen. Im in die Hornhaut 16 eingesetzten Zustand weisen die Sets 20, 21 dieser Ausgestaltung jeweils einen Z-förmigen Querschnitt auf.

Der durch das jeweils in eine distale Ebene eingesetzte Implantatsglied 22, 24 gebildete Schenkel der Z-Form ist dabei in Richtung der Verformung 17 gerichtet. Dagegen weist der durch das jeweils in eine proximale Ebene eingesetzte Implantatsglied 23, 25 gebildete Schenkel der Z-Form von der Verformung 17 weg.

Die Sets 20, 21 werden derart unter Spannung in die Hornhaut 16 eingesetzt, dass die distalen Implantatsglieder 22, 24 eine Druckkraft auf die Verformung 17 ausüben, während die proximalen Implantatsglieder 23, 25 eine Zugkraft auf die Verformung ausüben. Dies ist in Figur 3 jeweils durch die Pfeile 28, 29, 30, 31 veranschaulicht. Auf diese Weise ist es möglich, mit einem einstückigen Set 20, 21 sowohl eine Zugkraft als auch eine Druckkraft auf die lokale Verformung 17 auszuüben und die Verformung so besonders wirkungsvoll auszugleichen.

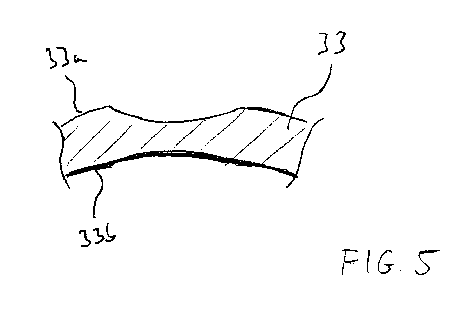

Während in den Figuren 1 und 3 die lokale Verformung der Hornhaut jeweils im noch nicht ausgeglichenen Zustand dargestellt ist, ist in den Figuren 4 und 5 jeweils ein ausschnittsweiser Querschnitt der Hornhaut 32, 33 eines menschlichen Auges dargestellt, wobei jeweils der Zustand der Hornhaut 32, 33 gezeigt ist, in dem eine vorher bestehende Hornhautverformung bereits ausgeglichen ist. Die Hornhaut 32, 33 weist jeweils eine distale Oberfläche 32a, 33a und eine der Netzhaut des Auges zugewandte proximale Oberfläche 32b, 33b auf.

Je nach Ausbildung der Sets zum Einsetzen in die Hornhaut 32, 33 und der dafür vorgesehenen Aufnahmen in der Hornhaut lässt sich die Verformung beispielsweise derart korrigieren, dass im ausgeglichenen Zustand die distale Oberfläche der Verformung an die distale Oberfläche 32a der gesunden Hornhaut 32 angepasst ist. Dies ist in Figur 4 dargestellt. Dieser Zustand wird insbesondere erreicht, wenn in einer distalen Ebene der Hornhaut 32 vorgesehene Implantatsglieder nur eine geringe Druckkraft auf die Verformung ausüben, während in einer proximalen Ebene der Hornhaut 32 vorgesehene Implantatsglieder eine dominierende Zugkraft auf die Verformung ausüben. In diesem Fall kann eine geringfügige Verformung im Bereich der proximalen Oberfläche 32b der Hornhaut auch nach dem Ausgleich zurückbleiben, die in Bezug auf die Sehkraft aber keine wesentlichen Nachteile bewirkt.

Es ist alternativ auch möglich, die proximale Oberfläche der Verformung an die proximale Oberfläche 33b der gesunden Hornhaut 33 anzupassen. Dies stellt den stabilsten Zustand der ausgeglichenen Verformung dar. Dieser Zustand ist in Figur 5 dargestellt. Dabei kann es je nach Stärke der Verformung zu einer geringfügigen Einwölbung der distalen Oberfläche 33a der Hornhaut 33 in dem Bereich der vorherigen Verformung kommen, die mit einer entsprechenden Linse ausgeglichen werden kann. Erreicht wird der in Figur 5 dargestellte Ausgleich insbesondere, indem in einer distalen Ebene der Hornhaut 33 durch ein entsprechend ausgebildetes Implantatsglied eine stärkere Druckkraft ausgeübt wird.

### BEZUGSZEICHENLISTE

- 1: Hornhaut
- 1a: distale Oberfläche der Hornhaut
- 1b: proximale Oberfläche der Hornhaut
- 2: lokale Verformung der Hornhaut
- 3: Aufnahme
- 4: Aufnahme
- 5: Implantatsglied
- 6: Implantatsglied
- 7: Pfeil
- 8: Pfeil
- 9: Entlastungskammer
- 10: Implantatsglied
- 11: Aufnahme
- 12: Pfeil
- 13: Pfeil
- 14: Pfeil
- 15: magnetisch manipulierbarer Bereich
- 16: Hornhaut
- 16a: distale Oberfläche der Hornhaut
- 16b: proximale Oberfläche der Hornhaut
- 17: lokale Verformung der Hornhaut
- 18: Aufnahme
- 19: Aufnahme
- 20: Set
- 21: Set
- 22: Implantatsglied
- 23: Implantatsglied
- 24: Implantatsglied
- 25: Implantatsglied
- 26: Verbindungssteg
- 27: Verbindungssteg
- 28: Pfeil
- 29: Pfeil
- 30: Pfeil
- 31: Pfeil
- 32: Hornhaut
- 32a: distale Oberfläche der Hornhaut
- 32b: proximale Oberfläche der Hornhaut
- 33: Hornhaut
- 33a: distale Oberfläche der Hornhaut
- 33b: proximale Oberfläche der Hornhaut

## Patentansprüche

1. Einrichtung zum Ausgleich einer lokalen Verformung (2,17) der Hornhaut (1, 16, 32, 33) eines Auges, mit einem mindestens zwei Implantatsglieder (5,6,10,22,23,24,25) umfassenden Set (20,21) zum Einsetzen in mindestens eine in die Hornhaut (1,16,32,33) eingebrachte Aufnahme (3,4,11,18,19), **dadurch gekennzeichnet, dass** mindestens zwei Implantatsglieder (5, 6, 10, 22, 23, 24, 25) in unterschiedlichen übereinander liegenden Ebenen angeordnet sind.

2. Einrichtung nach Anspruch 1, **dadurch gekennzeichnet, dass** die Implantatsglieder derart ausgebildet sind, dass im in die Hornhaut (1,16,32,33) eingesetzten Zustand mindestens ein Implantatsglied (5, 6, 10, 22, 23, 24, 25) eine Druckkraft und mindestens ein Implantatsglied (5, 6, 10, 22, 23, 24, 25) eine Zugkraft auf den Bereich der lokalen Verformung (2,17) ausübt.

3. Einrichtung nach Anspruch 2, **dadurch gekennzeichnet, dass** das die Druckkraft ausübende Implantatsglied (5,6,10,22,23,24,25) zum Einsetzen in eine distale Ebene der Hornhaut (1,16,32,33) vorgesehen ist, und das die Zugkraft ausübende Implantatsglied (5,6,10,22,23,24,25) zum Einsetzen in eine proximale Ebene der Hornhaut (1,16,32,33) vorgesehen ist.

4. Einrichtung nach einem der Ansprüche 1 bis 3, **dadurch gekennzeichnet, dass** mindestens eines der Implantatsglieder (5, 6, 10, 22, 23, 24, 25) in Richtung seiner Längserstreckung bogenförmig ausgebildet ist.

5. Einrichtung nach Anspruch 4, **dadurch gekennzeichnet, dass** das Implantatsglied (5, 6, 10, 22, 23, 24, 25) einen Winkelbereich von weniger als 360° umfasst.

6. Einrichtung nach Anspruch 5, **dadurch gekennzeichnet, dass** das Implantatsglied (5, 6, 10, 22, 23, 24, 25) einen Winkelbereich von weniger als 180° umfasst.

7. Einrichtung nach einem der Ansprüche 4 bis 6, **dadurch gekennzeichnet, dass** das bogenförmige Implantatsglied (5, 6, 10, 22, 23, 24, 25) eine zweite Krümmung aufweist, deren Krümmungsvektor im Wesentlichen senkrecht zu dem Krümmungsvektor der Bogenkrümmung steht.

8. Einrichtung nach einem der voranstehenden Ansprüche, **dadurch gekennzeichnet, dass** mindestens eines der Implantatsglieder (5,6,10,22,23,24,25) mäanderförmig ausgebildet ist.

9. Einrichtung nach einem der Ansprüche 1 bis 7, **dadurch gekennzeichnet, dass** mindestens eines der Implantatsglieder (5,6,10,22,23,24,25) polygonförmig ausgebildet ist.

10. Einrichtung nach einem der voranstehenden Ansprüche, **dadurch gekennzeichnet, dass** mindestens eines der Implantatsglieder (5,6,10,22,23,24,25) entlang seiner Längserstreckung unterschiedliche Festigkeits- und/oder Elastizitätswerte aufweist.

11. Einrichtung nach einem der voranstehenden Ansprüche, **dadurch gekennzeichnet, dass** mindestens eines der Implantatsglieder (5,6,10,22,23,24,25) einen keilförmigen Querschnitt aufweist.

12. Einrichtung nach einem der voranstehenden Ansprüche, **dadurch gekennzeichnet, dass** das Set (20,21) einstückig ausgebildet ist.

13. Einrichtung nach Anspruch 12, **dadurch gekennzeichnet, dass** die Implantatsglieder (5,6,10,22,23,24,25) im Wesentlichen parallel zueinander ausgerichtet sind und durch einen Verbindungssteg (26,27) miteinander verbunden sind, wobei der Verbindungssteg (26,27) mit seinem einen Stegende mit einem in Richtung der Quererstreckung der Implantatsglieder (5, 6, 10, 22, 23, 24, 25) vorderen Ende des einen Implantatsglieds (5,6,10,22,23,24,25) verbunden ist und mit seinem anderen Stegende mit einem in Richtung der Quererstreckung der Implantatsglieder (5, 6, 10, 22, 23, 24, 25) hinteren Ende des anderen Implantatsglieds (5,6,10,22,23,24,25) verbunden ist.

14. Einrichtung nach einem der voranstehenden Ansprüche, **dadurch gekennzeichnet, dass** mindestens eines der Implantatsglieder (5,6,10,22,23,24,25) einen Bereich (15) aufweist, der mittels magnetischer Kräfte manipuliert werden kann.

15. Vorrichtung zum Ausgleich einer lokalen Verformung (2,17) der Hornhaut (1,16,32,33) eines Auges,
- mit einer Einrichtung zum Erfassen von Eigenschaftsdaten der lokalen Verformung (2,17),
- mit einer Einrichtung zum Simulieren des Ausgleichs der Verformung (2,17) der Hornhaut (1,16,32,33), mit der Maßgabe, dass mindestens zwei Implantatsglieder (5, 6, 10, 22, 23, 24, 25) in mindestens eine in die Hornhaut (1,16,32,33) eingebrachte Aufnahme (3,4,11,18,19) eingesetzt werden, wobei im in die Hornhaut (1,16,32,33) eingesetzten Zustand ein Implantatsglied (5,6,10,22,23,24,25) eine Zugkraft und ein Implantatsglied (5, 6, 10, 22, 23, 24, 25) eine Druckkraft auf den Bereich der lokalen Verformung (2,17) ausübt,
- mit einer Einrichtung zum Auswählen einer geeigneten Kombination einer in die Hornhaut (1,16,32,33) einzubringenden Aufnahme (3,4,11,18,19) und einer in die Aufnahme (3,4,11,18,19) einzusetzenden, gemäß einem der Ansprüche 1 bis 14 ausgebildeten Einrichtung, unter Berücksichtigung der Ergebnisse der Simulation, und
- mit einer Einrichtung zum Einbringen der Aufnahme (3,4,11,18,19) in die Hornhaut (1,16,32,33).

16. Vorrichtung nach Anspruch 15, **dadurch gekennzeichnet, dass** die Eigenschaftsdaten Daten bezüglich der Hornhautdicke,
- elastizität, -duktilität und/oder -festigkeit in dem verformten Bereich und/oder Daten bezüglich der Geometrie und/oder der Position der lokalen Verformung (2,17) sind.

17. Vorrichtung nach einem der Ansprüche 15 oder 16, **dadurch gekennzeichnet, dass** eine Einrichtung zum Erfassen der Eigenschaftsdaten des nicht verformten Bereichs der Hornhaut (1,16,32,33) vorgesehen ist.

18. Vorrichtung nach einem der Ansprüche 15 bis 17, **dadurch gekennzeichnet, dass** eine Einrichtung zum Markieren des für das Einbringen der Aufnahme (3,4,11,18,19) vorgesehenen Bereichs der Hornhaut (1,16,32,33) vor dem Einbringen der Aufnahme (3,4,11,18,19) vorgesehen ist.

19. Vorrichtung nach einem der Ansprüche 15 bis 18, **dadurch gekennzeichnet, dass** die Einrichtung zum Einbringen der Aufnahme (3,4,11,18,19) einen auf das Auge aufzusetzenden Adapter zum Fixieren des Auges während des Einbringens der Aufnahme (3,4,11,18,19) aufweist.

20. Vorrichtung nach einem der Ansprüche 15 bis 19, **dadurch gekennzeichnet, dass** die Einrichtung zum Einbringen der Aufnahme (3,4,11,18,19) in die Hornhaut (1,16,32,33) einen Laser, insbesondere einen Femtosekundenlaser, umfasst.

## Claims

1. Device for correcting a local deformation (2, 17) of the cornea (1, 16, 32, 33) of an eye, with a set (20, 21) comprising at least two implant members (5, 6, 10, 22, 23, 24, 25) for insertion into at least one receptacle (3, 4, 11, 18, 19) introduced into the cornea (1, 16, 32, 33), **characterised in that** at least two implant members (5, 6, 10, 22, 23, 24, 25) are arranged in different planes situated one above the other.

2. Device according to claim 1, **characterised in that** the implant members are so designed that, in the state where they are inserted into the cornea (1, 16, 32, 33), on the region of the local deformation (2, 17) at least one implant member (5, 6, 10, 22, 23, 24, 25) exerts a compressive force and at least one implant member (5, 6, 10, 22, 23, 24, 25) exerts a tensile force.

3. Device according to claim 2, **characterised in that** the implant member (5, 6, 10, 22, 23, 24, 25) which exerts the compressive force is intended for insertion in a distal plane of the cornea (1, 16, 32, 33) and the implant member (5, 6, 10, 22, 23, 24, 25) which exerts the tensile force is intended for insertion in a proximal plane of the cornea (1, 16, 32, 33).

4. Device according to one of claims 1 to 3, **characterised in that** at least one of the implant members (5, 6, 10, 22, 23, 24, 25) is formed arc-shaped in the direction of its longitudinal extent.

5. Device according to claim 4, **characterised in that** the implant member (5, 6, 10, 22, 23, 24, 25) covers an angular range of less than 360°.

6. Device according to claim 5, **characterised in that** the implant member (5, 6, 10, 22, 23, 24, 25) covers an angular range of less than 180°.

7. Device according to one of claims 4 to 6, **characterised in that** the arc-shaped implant member (5, 6, 10, 22, 23, 24, 25) has a second curvature whose curvature vector is substantially perpendicular to the curvature vector of the curvature of the arc.

8. Device according to one of the preceding claims, **characterised in that** at least one of the implant members (5, 6, 10, 22, 23, 24, 25) is of a meandering form.

9. Device according to one of claims 1 to 7, **characterised in that** at least one of the implant members (5, 6, 10, 22, 23, 24, 25) is of a polygonal form.

10. Device according to one of the preceding claims, **characterised in that** at least one of the implant members (5, 6, 10, 22, 23, 24, 25) has different levels of strength and/or elasticity along its longitudinal extent.

11. Device according to one of the preceding claims, **characterised in that** at least one of the implant members (5, 6, 10, 22, 23, 24, 25) has a wedge-shaped cross-section.

12. Device according to one of the preceding claims, **characterised in that** the set (20, 21) is monolithic.

13. Device according to claim 12, **characterised in that** the implant members (5, 6, 10, 22, 23, 24, 25) are aligned substantially parallel to one another and are connected together by a connecting web (26, 27), the connecting web (26, 27) being connected, by its one web end, to an end of the one implant member (5, 6, 10, 22, 23, 24, 25) which is at the front in the direction of the transverse extent of the implant members (5, 6, 10, 22, 23, 24, 25) and being connected, by its other web end, to an end of the other implant member (5, 6, 10, 22, 23, 24, 25) which is at the rear in the direction of the transverse extent of the implant members (5, 6, 10, 22, 23, 24, 25).

14. Device according to one of preceding claims, **characterised in that** at least one of the implant members (5, 6, 10, 22, 23, 24, 25) has a region (15) which can be manipulated by means of magnetic forces.

15. Apparatus for correcting a local deformation (2, 17) of the cornea (1, 16, 32, 33) of an eye,
- with a device for sensing data on the properties of the local deformation (2, 17),
- with a device for simulating the correction of the deformation (2, 17) of the cornea (1, 16, 32, 33), with the proviso that at least two implant members (5, 6, 10, 22, 23, 24, 25) are inserted into at least one receptacle (3, 4, 11, 18, 19) introduced into the cornea (1, 16, 32, 33), wherein, in the state inserted into the cornea, one implant member (5, 6, 10, 22, 23, 24, 25) exerts a tensile force on the region of the local deformation (2, 17) and one implant member (5, 6, 10, 22, 23, 24, 25) exerting a compressive force thereon,
- with a device for selecting, while taking into account of the results of the simulation, a suitable combination of a receptacle (3, 4, 11, 18, 19) to be introduced into the cornea (1, 18, 32, 33) and a device, formed in accordance with one of claims 1 to 14, to be inserted into the receptacle (3, 4, 11, 18, 19), and
- with a device for introducing the receptacle (3, 4, 11, 18, 19) into the cornea (1, 16, 32, 33).

16. Apparatus according to claim 15, **characterised in that** the data on the properties is data relating to the thickness, elasticity, ductility and/or strength of the cornea in the deformed region and/or data relating to the geometry and/or position of the local deformation (2, 17).

17. Apparatus according to either of claims 15 and 16, **characterised in that** a device is provided for sensing data on the properties of the non-deformed region of the cornea (1, 16, 32, 33).

18. Apparatus according to one of claims 15 to 17, **characterised in that** a device is provided for marking, before the introduction of the receptacle (3, 4, 11, 18, 19), the region of the cornea (1, 16, 32, 33) which is intended for the introduction of the receptacle (3, 4, 11, 18, 19).

19. Apparatus according to one of claims 15 to 18, **characterised in that** the device for introducing the receptacle (3, 4, 11, 18, 19) has an adapter to be placed onto the eye for fixing the eye in position during the introduction of the receptacle (3, 4, 11, 18, 19).

20. Apparatus according to one of claims 15 to 19, **characterised in that** the device for introducing the receptacle (3, 4, 11, 18, 19) into the cornea (1, 16, 32, 33) comprises a laser, in particular a femtosecond laser.

## Revendications

1. Dispositif pour corriger une déformation locale (2, 17) de la cornée (1, 16, 32, 33) d'un oeil, avec un set (20, 21) comprenant au moins deux membres d'implant (5, 6, 10, 22, 23, 24, 25) pour l'insertion dans au moins un réceptacle (3, 4, 11, 18, 19) introduit dans la cornée (1, 16, 32, 33), **caractérisé en ce qu'**au moins deux membres d'implant (5, 6, 10, 22, 23, 24, 25) sont disposés dans des plans différents situés l'un au-dessus de l'autre.

2. Dispositif selon la revendication 1, **caractérisé en ce que** les membres d'implant sont conçus de telle manière à ce que, dans l'état dans lequel ils sont insérés dans la cornée (1, 16, 32, 33), sur la région de la déformation locale (2, 17) au moins un membre d'implant (5, 6, 10, 22, 23, 24, 25) exerce une force de compression et au moins un membre d'implant (5, 6, 10, 22, 23, 24, 25) exerce une force de tension.

3. Dispositif selon la revendication 2, **caractérisé en ce que** le membre d'implant (5, 6, 10, 22, 23, 24, 25) qui exerce la force de compression est destiné à l'insertion dans un plan distal de la cornée (1, 18, 32, 33) et le membre d'implant (5, 6, 10, 22, 23, 24, 25) qui exerce la force de tension est destiné à l'insertion dans un plan proximal de la cornée (1, 16, 32, 33).

4. Dispositif selon l'une des revendications 1 à 3, **caractérisé en ce qu'**au moins un des membres d'implant (5, 6, 10, 22, 23, 24, 25) est fait en forme d'arc dans la direction de son extension longitudinale.

5. Dispositif selon la revendication 4, **caractérisé en ce que** le membre d'implant (5, 6, 10, 22, 23, 24, 25) couvre une plage angulaire de moins de 360°.

6. Dispositif selon la revendication 5, **caractérisé en ce que** le membre d'implant (5, 6, 10, 22, 23, 24, 25) couvre une plage angulaire de moins de 180°.

7. Dispositif selon l'une des revendications 4 à 6, **caractérisé en ce que** le membre d'implant en forme d'arc (5, 6, 10, 22, 23, 24, 25) possède une seconde courbure dont le vecteur de courbure est substantiellement perpendiculaire au vecteur de courbure la courbure de l'arc.

8. Dispositif selon l'une des revendications précédentes, **caractérisé en ce qu'**au moins un des membres d'implant (5, 6, 10, 22, 23, 24, 25) est de forme de méandre.

9. Dispositif selon l'une des revendications 1 à 7, **caractérisé en ce qu'**au moins un des membres d'implant (5, 6, 10, 22, 23, 24, 25) est de forme polygonale.

10. Dispositif selon l'une des revendications précédentes, **caractérisé en ce qu'**au moins un des éléments d'implant (5, 6, 10, 22, 23, 24, 25) possède différents niveaux de solidité et/ou d'élasticité le long de son extension longitudinale.

11. Dispositif selon l'une des revendications précédentes, **caractérisé en ce qu'**au moins un des membres d'implant (5, 6, 10, 22, 23, 24, 25) a une section transversale en forme de coin.

12. Dispositif selon l'une des revendications précédentes, **caractérisé en ce que** le set (20, 21) est monolithique.

13. Dispositif selon la revendication 12, **caractérisé en ce que** les membres d'implant (5, 6, 10, 22, 23, 24, 25) sont alignés substantiellement en parallèle les un à l'autre et sont connectés par une âme de connexion (26, 27), l'âme de connexion (26, 27) étant connectée, par l'une de ses extrémités d'âme, à une extrémité de l'un membre d'implant (5, 6, 10, 22, 23, 24, 25) qui est à l'avant dans la direction de l'extension transversale des membres d'implant (5, 6, 10, 22, 23, 24, 25) et étant connectée, par l'autre de ses extrémités d'âme, à une extrémité de l'autre membre d'implant (5, 6, 10, 22, 23, 24, 25) qui est à l'arrière dans la direction de l'extension transversale des membres d'implant (5, 6, 10, 22, 23, 24, 25).

14. Dispositif selon l'une des revendications précédentes, **caractérisé en ce qu'**au moins un des membres d'implant (5, 6, 10, 22, 23, 24, 25) possède une région (15) qui peut être manipulée au moyen de forces magnétiques.

15. Appareil pour corriger une déformation locale (2, 17) de la cornée (1, 16, 32, 33) d'un oeil,
- avec un dispositif pour détecter des données sur les propriétés de la déformation locale (2, 17),
- avec un dispositif pour simuler la correction de la déformation (2, 17) de la cornée (1, 16, 32, 33), à condition qu'au moins deux membres d'implant (5, 6, 10, 22, 23, 24, 25) soient insérés dans au moins un réceptacle (3, 4, 11, 18, 19) introduit dans la cornée (1, 16, 32, 33), où, dans l'état dans inséré dans la cornée, sur la région de la déformation locale (2, 17) un membre d'implant (5, 6, 10, 22, 23, 24, 25) exerce une force de tension et un membre d'implant (5, 6, 10, 22, 23, 24, 25) exerce une force de compression,
- avec un dispositif pour sélectionner, tout en tenant compte des résultats de la simulation, une combinaison adaptée d'un réceptacle (3, 4, 11, 18, 19) à introduire dans la cornée (1, 16, 32, 33) et un dispositif, formé selon l'une des revendications 1 à 14, à insérer dans le réceptacle (3, 4, 11, 18, 19), et
- avec un dispositif pour introduire le réceptacle (3, 4, 11, 18, 19) dans la cornée (1, 16, 32, 33).

16. Appareil selon la revendication 15, **caractérisé en ce que** les données sur les propriétés sont des données se rapportant à l'épaisseur, l'élasticité, la ductilité et/ou la solidité de la cornée dans la région déformée et/ou des données se rapportant à la géométrie et/ou la position de la déformation locale (2, 17).

17. Appareil selon l'une ou l'autre des revendications 15 et 16, **caractérisé en ce qu'**un dispositif est prévu pour détecter des données sur les propriétés de la région non déformée de la cornée (1, 16, 32, 33).

18. Appareil selon l'une des revendications 15 à 17, **caractérisé en ce qu'**un dispositif est prévu pour marquer, avant l'introduction du réceptacle (3, 4, 11, 18, 19), la région de la cornée (1, 16, 32, 33) qui est destinée à l'introduction du réceptacle (3, 4, 11, 18, 19).

19. Appareil selon l'une des revendications 15 à 18, **caractérisé en ce que** le dispositif pour introduire le réceptacle (3, 4, 11, 18, 19) possède un adaptateur à placer sur l'oeil pour fixer l'oeil en position pendant l'introduction du réceptacle (3, 4, 11, 18, 19).

20. Appareil selon l'une des revendications 15 à 19, **caractérisé en ce que** le dispositif pour introduire le réceptacle (3, 4, 11, 18, 19) dans la cornée (1, 16, 32, 33) comprend un laser, en particulier un laser femtoseconde.
